# EUROPÄISCHE PATENTSCHRIFT

(11) **EP 0 591 106 B1**
(45) Veröffentlichungstag und Bekanntmachung des Hinweises auf die Patenterteilung: **14.01.1998**
(21) Anmeldenummer: 93810668.9
(22) Anmeldetag: 21.09.1993
(51) Int. Cl.: C09B 67/42, C07D 493/10, B41M 5/30

(54) **Konzentrierte, wässrige Flüssigformulierungen von Farbbildnern**
Concentrated aqueous liquid compositions of colour formers
Compositions liquides aqueuses concentrées de formateurs de couleurs

(30) Priorität: 30.09.1992 CH 3047/92
(43) Veröffentlichungstag der Anmeldung: 06.04.1994
(73) Patentinhaber: Ciba Specialty Chemicals Holding Inc., 4057 Basel (CH)
(72) Erfinder: Scartazzini, Roger, Dr., CH-4304 Giebenach (CH); Huber, Klaus, Dr., D-79110 Freiburg (DE)

(56) Entgegenhaltungen:
- EP-A- 0 266 311
- EP-A- 0 331 636
- EP-A- 0 333 649
- EP-A- 0 384 895
- EP-A- 0 561 738
- CH-A- 567 077

## Beschreibung

Die vorliegende Erfindung betrifft konzentrierte, wässrige Flüssigformulierungen von Farbbildnern, ein Verfahren zur Herstellung dieser Formulierungen sowie die Verwendung dieser Formulierungen in wärmeempfindlichen Aufzeichnungsmaterialien.

Konzentrierte, wässrige Flüssigformulierungen von Farbbildnern für wärmempfindliche Aufzeichnungsmaterialien sind bekannt, z.B. aus der US-A-4,363,664. Es handelt sich dabei um wässrige Dispersionszusammensetzungen aus einem Farbbildner und einem oberflächenaktiven Mittel. Überraschenderweise wurde nun gefunden, dass Flüssigformulierungen, die neben einem oder mehreren Farbbildnern und gewissen anionischen Tensiden zusätzlich ein Verdickungsmittel enthalten, eine ausgezeichnete Lagerstabilität aufweisen.

Gegenstand der vorliegenden Erfindung ist daher eine Flüssigformulierung enthaltend
a) als Farbbildner eine Fluoranverbindung der Formel 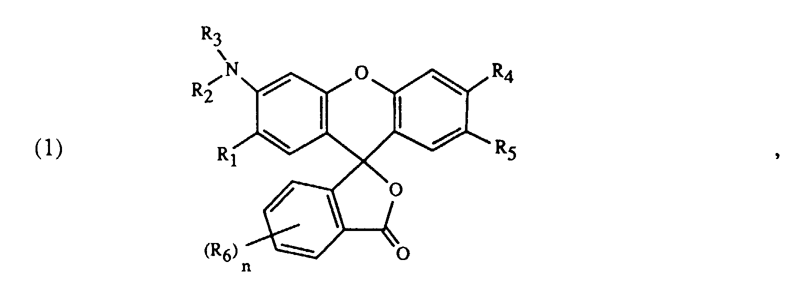 worin
   - R₁: Wasserstoff oder C₁-C₄-Alkyl;
   - R₂ und R₃: unabhängig voneinander Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-AIkoxy-C₁-C₄Alkyl; 2-Tetrahydrofuranyl, oder
   - R₂ und R₃: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
   - R₄: Wasserstoff, Hydroxy oder C₁-C₄-Alkyl;
   - R₅: Nitro; SO₂R₇; SO₂OR₈; SO₂NR₉R₁₀; COR₁₁; CONR₉R₁₀; oder C₁-C₄-Halogenalkyl; nicht substituierter oder durch Halogen, C₁-C₄-Alkyl, C₁-C₄-Halogenalkyl oder C₁-C₄-Alkoxy substituiertes C₆-C₁₀-Aryl; nicht substituierter oder durch Halogen oder Hydroxy substituierter 2-Triazinyl- oder 1-Benztriazolylrest;
   - R₆: Halogen; Nitro; C₁-C₄-Alkyl; C₁-C₄-Halogenalkyl; Amino; mono-C₁-C₄-Alkylamino; di-C₁-C₄-Alkylamino; oder COR₁₁;
   - n: 0; 1; 2; 3; oder 4;
   - R₇: C₁-C₈-Alkyl; oder C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-Alkyl;
   - R₈: Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
   - R₉ und R₁₀: unabhängig voneinander Wasserstoff; oder C₁-C₈-Alkyl; oder
   - R₉ und R₁₀: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring; und
   - R₁₁: Wasserstoff; Hydroxy; C₁-C₈-Alkyl; C₁-C₈-Alkoxy; C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy
   bedeutet,
(b) als anionische Tenside
   (ba) sulfatierte Alkane
   (bb) Alkansulfonate
   (bc) sulfonierte Carboxylate oder
   (bd) sulfatierte Carboxylate, oder
   (be) sauer Ester oder deren Salze von Alkylenoxidaddukten und
(c) ein Verdickungsmittel.

In der erfindungsgmässen Zusammensetzung können die Komponenten (a), (b) und (c) sowohl als Einzelverbindungen oder als Gemisch mehrerer Verbindungen vorliegen.

In der Literatur werden die einzelnen Substituentenposititionen im Fluoranring unterschiedlich numeriert. In der vorliegenden Anmeldung wird die folgende Numerierung verwendet:

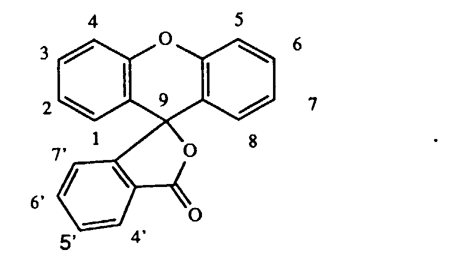

Im Rahmen der vorstehenden Definition haben die jeweiligen Substituenten oder Radikale insbesondere die folgenden Bedeutungen:

Halogen ist Fluor, Chlor oder Brom, insbesondere Fluor oder Chlor.

Alkyl bedeutet im Rahmen der jeweiligen Definition geradkettiges oder verzweigtes Alkyl. Beispiele solcher Alkylreste sind Methyl, Ethyl, n-Propyl, Isopropyl, n-Butyl, 1-Methylbutyl, sek.Butyl, tert.Butyl, n-Pentyl, Amyl, Isoamyl, n-Hexyl, 2-Ethyl-hexyl, n-Heptyl, n-Octyl, Isooctyl, 1,1,3,3-Tetramethylbutyl.

Beispiele für Halogenalkyl sind insbesondere die C₁-C₂-Halogenalkylreste, wie Trichlormethyl, Trifluormethyl, Dichlorfluormethyl, Difluorchlormethyl, Perchlorethyl, 1,1,2,2-Tetrachlorethyl, 1,1,2,2-Tetrafluorethyl, 2,2,2-Trichlorethyl. R₅ als C₁-C₈-Halogenalkyl sind insbesondere die zuvor genannten Halogenalkyle aber auch Alkylradikale, bei denen alle oder zumindest der überwiegende Teil der C-H Bindungen durch C-Cl oder C-F ersetzt ist.

Alkoxy ist insbesondere Methoxy, Ethoxy, n-Propoxy, Isopropoxy, n-Butoxy, Isobutoxy, sek.Butoxy und tert.Butoxy, C₁-C₄Alkoxy-C₁-C₄Alkyl insbesondere Methoxymethyl, Methoxyethyl, Ethoxymethyl oder Ethoxyethyl.

Mono-C₁-C₅-Alkylamino ist insbesondere Methylamino, Ethylamino, Propylamino, Butylamino und Pentylamino. Di-C₁-C₅-Alkylamino umfaßt sowohl die gemischten wie auch die gleichnamig substituierten Radikale, wie Methylethylamino, Dimethylamino, Diethylamino, Methylpropylamino, Methylbutylamino, Di-n-propylamino, Di-isopropyl-amino, Di-n-butylamino und Di-n-pentylamino etc..

In Phenyl-C₁-C₄-Alkyl und Phenyl-C₁-C₄-Alkoxy kann der Phenylrest über eine geradkettige oder verzweigte Alkyl- bzw. Alkoxykette gebunden sein. Bevorzugt ist Phenethyl, Benzyl und Phenylmethoxy.

Der Phenylrest in Phenyl-C₁-C₄-Alkyl, Phenyl-C₁-C₄-Alkoxy bzw. Phenyl ist vorzugweise unsubstituiert oder bis zu dreifach gleich oder verschieden durch die genannten Substituenten substituiert.

Beispiele für C₃-C₅-Alkenyl sind Allyl, 1-Propenyl oder 2-Pentenyl, Isopropenyl oder 2-Butenyl. Allyl ist bevorzugt. C₄-C₇-Cyclohexyl bedeutet Cyclobutyl, Cyclopentyl, Cyclohexyl oder Cycloheptyl, wobei Cyclohexyl im Vordergrund des Interesses steht.

C₆-C₁₀-Aryl bedeutet Phenyl oder Naphthyl.

Bevorzugt sind Fluorane der Formel (1), worin
- R₁: Wasserstoff oder C₁-C₄-Alkyl;
- R₂ und R₃: unabhängig voneinander Wasserstoff; C₁-C₅-Alkyl; oder
- R₂ und R₃: gemeinsam mit dem Stickstoffatom, an welches sie gebunden sind, einen unsubstituierten oder durch C₁-C₄-Alkyl substituierten Pyrrolidin- oder Piperidin-Ring;
- R₄: Wasserstoff oder C₁-C₄-Alkyl;
- R₅: Nitro; SO₂R₇; SO₂OR₈; SO₂NR₉R₁₀; COR₁₁; CONR₉R₁₀; C₁-C₄-Halogenalkyl; unsubstituiertes oder im Phenylrest durch C₁-C₄-Alkyl; C₁-C₄-Halogenalkyl, C₁-C₄-Alkoxy substituiertes Phenyl; Phenylamino; oder Phenyl-C₁-C₄-Alkylamino;
- n: 0; 1; 2; 3; oder 4;
- R₆: wenn n 1, 2, 3 oder 4 ist; Halogen; wenn n 1 oder 2 ist C₁-C₄-Alkyl; C₁-C₄-Halogenalkyl; oder wenn n 1 ist, Nitro, COR₁₁; Amino, mono-C₁-C₄-Alkylamino; oder di-C₁-C₄-Alkylamino;
- R₇: C₁-C₄-Alkyl; oder C₁-C₄-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₂-Alkyl;
- R₈: Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₂-alkyl;
- R₉ und R₁₀: unabhängig voneinander Wasserstoff; oder C₁-C₈-Alkyl; oder
- R₉ und R₁₀: gemeinsam mit dem Stickstoffatom, an welches sie gebunden sind, einen unsubstituierten oder durch C₁-C₄-Alkyl substituierten Pyrrolidin- oder Piperidin-Ring; und
- R₁₁: Wasserstoff; C₁-C₄-Alkyl; C₁-C₄-Halogenalkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy
bedeutet,

Hervorzuheben sind Farbbildner der Formel (1)
worin
- R₁: Wasserstoff oder Methyl;
- R₂ und R₃: unabhängig voneinander Wasserstoff; C₁-C₅-Alkyl; oder
- R₂ und R₃: gemeinsam mit dem Stickstoffatom, an welches sie gebunden sind, einen unsubstituierten Pyrrolidin- oder Piperidin-Ring;
- R₄: Wasserstoff oder Methyl;
- R₅: Nitro; SO₂R₇; SO₂NR₉R₁₀; COR₁₁; CONR₉R₁₀; C₁-C₄-Halogenalkyl; unsubstituiertes oder im Phenylrest durch Halogen, C₁-C₄-Alkyl; C₁-C₄-Halogenalkyl oder C₁-C₄-Alkoxy substituiertes Phenyl; Phenyl-C₁-C₄-Alkyl; Phenylamino; oder Phenyl-C₁-C₄-Alkylamino;
- n: 0; 1; 2; 3; oder 4;
- R₆: wenn n 1, 2, 3 oder 4 ist; Halogen; wenn n 1 oder 2 ist Methyl; oder wenn n 1 ist, Nitro, Amino, mono-C₁-C₄-Alkylamino oder di-C₁-C₄-Alkylamino;
- R₇: C₁-C₄-Alkyl; oder C₁-C₄-Halogenalkyl; unsubstituiertes oder im Phenylrest durch Halogen, C₁-C₄-Alkyl oder C₁-C₄-Alkoxy substituiertes Phenyl;
- R₉ und R₁₀: unabhängig voneinander Wasserstoff; oder C₁-C₄-Alkyl;
- R₁₁: Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy
bedeutet,

Insbesondere betrifft die Erfindung Flüssigformulierungen, die Farbbildner der Formel (1) enthalten, worin R₅ SO₂R₇, SO₂OR₈, SO₂NR₉R₁₀, COR₁₁, CONR₉R₁₀, C₁-C₄-Halogenalkyl oder Nitro bedeutet.

Unter den genannten Verbindungen der Formel (1) sind diejenigen bevorzugt, bei denen R₅ COR₁₁ bedeutet und ganz besonders diejenigen, bei denen R₁₁ C₁-C₄-Alkyl, C₁-C₄-Alkoxy oder Phenyl-C₁-C₄-Alkoxy bedeutet. Im Vordergrund des Interesses stehen dabei Verbindungen, bei denen R₁₁ C₁-C₄-Alkoxy oder Phenyl-C₁-C₄-Alkoxy bedeutet.

Ganz besonders bevorzugt kommen als Komponente (a) Farbbildner der Formel (1) in Betracht, bei denen R₂ und R₃ unabhängig voneinander C₁-C₅-Alkyl, C₅-C₇-Cycloalkyl, Benzyl oder Allyl bedeuten.

Von ganz besonderem Interesse sind dabei Verbindungen der Formel (1), bei denen R₂ und R₃ unabhängig voneinander C₁-C₄-Alkyl bedeuten.

Weitere sehr wichtige Farbbildner sind solche der Formel (1), bei denen R₅ Phenyl; Phenylamino; oder Phenyl-C₁-C₄-Alkylamino bedeutet.

Bei der Komponente (ba) handelt es sich um Salze der Schwefelsäureester von Fettalkoholen der Formel

(2) R₁₂-O-SO₃-M₁,

worin R₁₂ einen Alkylrest von 6 bis 18 Kohlenstoffatomen und
M₁ Alkalimetall
bedeutet.

Der Alkylrest R₁₂ in dieser Formel ist verzweigt oder unverzweigt und leitet sich z.B. vom Hexanol, Heptanol sowie vom Octyl-, Nonyl-, Decyl- und Dodecylalkohol, ferner auch vom Lauryl-, Myristyl-, Cetyl- und Stearylalkohol ab.

M₁ bedeutet Lithium, Kalium oder Natrium. Kalium und Natrium sind bevorzugt.

Bevorzugt sind die Natriumsalze der Schwefelsäureester von Fettalkoholen mit 6 bis 12, insbesondere 8 oder 9 Kohlenstoffatomen. Besonders gute Ergebnisse werden mit den Schwefelsäureestern von verzweigten Isomeren der genannten Alkohole erzielt, wie z.B. 2-Ethylhexanol, Trimethylhexanol und Trimethylnonylalkohol. Die genannten Salze der Schwefelsäureester können allein oder als Gemisch untereinander in der erfindungsgemässen Formulierung verwendet werden.

Als Komponente (bb) kommen solche Verbindungen in Betracht, bei denen die Alkylkette 8 bis 20 Kohlenstoffatome aufweist. Bevorzugt sind dabei die sekundären Alkansulfonate (Paraffinsulfonate) mit einer Alkylkettenlänge von 14 bis 18 Kohlenstoffatomen, bei denen die Sulfonatgruppe statistisch in der Alkylkette angeordnet ist.

Bei den Verbindungen der Komponente (bc) handelt es sich um Sulfonate von Estern von Polycarbonsäuren wie z.B. der Malon- oder Bernsteinsäure. Es kommen dabei Diester, wie z.B. Sulfobernsteinsäuredioctylester oder Halbester wie Sulfobernsteinsäurehalbester, die auf C₁₂-C₂₄Fettalkoholen oder ethoxylierten Alkanolamiden basieren, in Betracht. Beispielhafte Verbindungen sind Dihexylsulfosuccinate, Di-2-ethylhexylsulfosuccinate, Dioctylsulfosuccinate oder Sulfosuccinamide. Ganz besonders geeignet sind Verbindungen der Formel 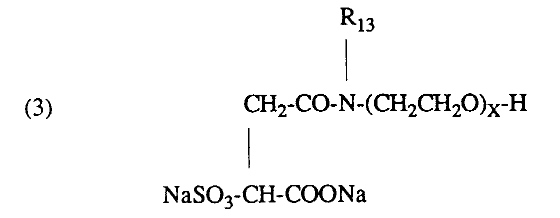 worin
- R₁₃: Wasserstoff oder (CH₂CH₂O)ₓH and
- x: 1 bis 10 bedeutet.

Bei den Verbindungen der Komponente (bd) handelt es sich um Alkalimetall-, Ammonium- oder Aminsalze der Schwefelsäureester von Fettsäuren mit 10 bis 22 Kohlenstoffatomen, z.B. um die Salze der Laurin-, Myristin-, Palmitin-, Stearin- oder Oelsäure oder Kolophoniumsalze. Besonders bevorzugt sind dabei das Kaliumsalz des Schwefelsäureesters einer C₁₂-C₁₈Fettsäure.

Als saure Ester oder deren Salze von Alkylenoxidaddukten (be) kommen Verbindungen der Formel 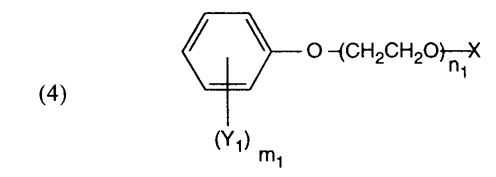 in Betracht, worin
Y₁ C₄-C₁₂-Alkyl, Phenyl, Tolyl, Phenyl-C₁-C₃-Alkyl oder Tolyl-C₁-C₃-Alkyl bedeutet,
X₁ ein Säurerest ist, der sich von Schwefelsäure oder Orthophosphorsäure ableitet, und
m₁ 1 bis 3 und n₁ 4 bis 40 sind. Ganz besonders interessante Verbindungen sind dabei Verbindungen der Formel 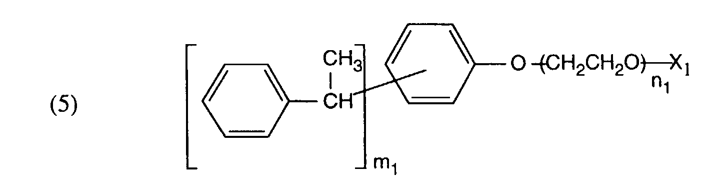
worin X₁, m₁ und n₁ die angegebene Bedeutungen haben.

Bei der erfindungsgemässen Formulierung wird als anionische Tenside (b) bevorzugt die Komponente (bc), (bd) oder (be) eingesetzt.

Als Verdickungsmittel (c) können folgende Verbindungen verwendet werden:
(ca) ionisch modifizierte Polysaccharide,
(cb) nichtionische Cellulose und deren Derivate,
(cc) synthetische, wasserlösliche Carboxylate,
(cd) Polyvinylalkohol oder
(ce) Bentonite und
(cf) nichtionogene Tenside.

Bei der Komponente (ca) handelt es sich um hochmolekulare Verbindungen, die als Grundbausteine Glucose, Mannuronsäure, Guluronsäure, Mannose, Galactose, Xylose, Arabinose, Fucose, D-Galactose oder L-Rhamnose enthalten. Es kommen sowohl Homo- als auch Heteropolysaccharide in Betracht, wobei sich die Homopolysaccharide aus einheitlichen Monosaccharideinheiten zusammensetzen , während die Heteropolysaccharide aus verschiedenen Grundbausteinen zusammengesetzt sein können. Beispiele für anionische Polysaccharide sind carboxymethylierter Guarether, Natrium-Carboxymethylcellulose (Na-CMC), Carboxymethylstärke, carboxymethyliertes Johannisbrotkernmehl, und ganz besonders bevorzugt Xanthan und Natriumalginat.

Bei der Komponente (cb) handelt es sich um nichtionische Cellulosederivate, die chemisch modifiziert sind, wie z.B. Celluloseester oder alkylierte Cellulose (Celluloseether), wie z.B. Methylcellulose, Hydroxyethylcellulose, Methylhydroxyethylcellulose, Hydroxypropylcellulose und Hydroxypropylmethylcellulose.

Bei den Verbindungen der Komponente (cc) handelt es sich in erster Linie um Alkalisalze von Homo- oder Copolymerisaten der Acrylsäure mit einem durchschnittlichen Molekulargewicht von 100 000 bis 300 000. Besonders bevorzugt ist Polyacrylsäure mit einem Molekulargewicht von 150,000 bis 200,000.

Der Polyvinylalkohol entsprechend der Komponente (cd) hat ein Molekulargewicht von 15,000 bis 1,500,000, vorzugsweise 60,000 bis 100,000 und einen Hydrolysierungsgrad von 80 bis 100 Mol%, vorzugsweise 85 bis 90 Mol%.

Als in Wasser schwer lösliche nichtionogene Tenside entsprechende Komponente (cf) kommen in Betracht
(cfa) Alkenoxiaddukte an
   - (α): gesättigte oder ungesättigte 1-6-wertige aliphatische Alkohole,
   - (β): Fettsäuren,
   - (γ): Fettamine,
   - (δ): Fettamide,
   - (ε): Diamine,
   - (ζ): Sorbitanester,
(cfb) Alkenoxid-Kondensationsprodukte (Blockpolymerisate),
(cfc) Polymerisate von Vinylpyrrolidon, Vinylacetat oder Vinylalkohol und
(cfd) Co- oder Ter-polymere von Vinylpyrrolidon mit Vinylacetat und/oder Vinylalkohol.

Die nichtionogene Komponente (cfa) ist vorteilhafterweise
- ein Alkylenoxidanlagerungsprodukt von 1 bis 100 Mol Alkylenoxid, z.B. Ethylenoxid und/oder Propylenoxid, an 1 Mol eines aliphatischen Monoalkohols mit mindestens 4 Kohlenstoffatomen, eines 3- bis 6-wertigen aliphatischen Alkohols oder eines gegebenenfalls durch Alkyl, Phenyl, α-Tolylethyl, Benzyl, α-Methylbenzyl oder α,α-Dimethylbenzyl substituierten Phenols;
- ein Alkylenoxidanlagerungsprodukt von 1 bis 100, vorzugsweise 2 bis 80 Mol Ethylenoxid wobei einzelne Ethylenoxideinheiten durch substituierte Epoxide, wie Styroloxid und/oder Propylenoxid, ersetzt sein können, an höhere unbesättigte oder gesättigte Monoalkohole (α), Fettsäuren (β), Fettamine (γ) oder Fettamide (δ) mit 8 bis 22 Kohlenstoffatomen;
- ein Alkylenoxidanlagerungsprodukt, vorzugsweise ein Ethylenoxid-Propylenoxid-Addukt an Ethylendiamin (ε);
- ein ethoxyliertes Sorbitanester mit langkettigen Estergruppen, wie z.B. Polyexyethylen-Sorbitanmonolaurat mit 4 bis 20 Ethylenoxideinheiten oder Polyexyethylen-Sorbitantrioleat mit 4 bis 20 Ethylenoxideinheiten (ζ).

Bevorzugte Komponenten (cfb) sind Ethylenoxidaddukte an Polypropylenoxid (sogenannte EO-PO-Blockpolymere) und Propylenoxidaddukte an Polyethylenoxid (sogenannte umgekehrte EO-PO-Blockpolymere).

Besonders bevorzugt sind Ethylenoxid-Propylenoxid-Copolymere mit Molekulargewichten der Polypropylenoxidbasis von 400 bis 4000 und einem Ethylenoxidgehalt im Gesamtmolekül von 30-80 %, insbesondere 60-80 %.

Bei der erfindungsgemässen Formulierung wird als Verdickungsmittel (c) bevorzugt ionisch modifiziertes Polysaccharid (Komponente (ca)) eingesetzt, wobei Natriumalginat und Xanthan im Vordergrund des Interesses stehen. Weitere wichtige Verdickungsmittel für die erfindungsgemässe wässrige Formulierung sind Polyvinylalkohole (Komponente (cd)) mit den oben angegebenen Molekulargewichten sowie die nichtionogenen Tenside entsprechend Komponente (cf).

Wichtige, erfindungsgemässe wässrige Flüssigformulierungen enthalten
(a) einen Farbbildner der Formel (1),
(b) ein Alkalimetallsalz des Schwefelsäureesters einer C₁₀-C₁₈Fettsäure und
(c) ein ionisch modifiziertes Polysaccharid.

Besonders wichtig sind dabei Flüssigformulierungen, die
(a) einen Farbbildner der Formel (1), worin R₂ und R₃ unabhängig voneinander C₁-C₄Alkyl,
(b) Das Natriumsalz des Schwefelsäureesters einer C₁₀-C₁₈Fettsäure und
(c) Natriumalginat enthalten

Weitere interessante Flüssigformulierungen enthalten
(a) einen Farbbildner der Formel (1),
(b) ein Alkalimetallsalz des Schwefelsäureesters einer C₁₀-C₁₈Fettsäure und
(c) ein in Wasser schwerlösliches nichtionogenes Tensid.

Weitere wichtige, erfindungsgemässe Flüssigformulierungen enthalten
(a) einen Farbbildner der Formel (1), worin R₂ und R₃ unabhängig voneinander C₁-C₄Alkyl,
(b) ein anionisches Tensid der Formel (3) und
(c) Natriumalginat.

Die erfindungsgemässe wässrige Flüssigformulierung enthält, bezogen auf das Gewicht der gesamten Zusammensetzung
20 bis 60, vorzugsweise 35 bis 45 Gew.% eines Farbbildners (a)
1 bis 15, vorzugsweise 3 bis 8 Gew.% eines anionischen Tensides (b),
0,1 bis 5, vorzugsweise 0,5 bis 2 Gew.% eines Verdickungsmittels (c) und ad 100 % Wasser.

Die Herstellung der erfindungsgemässen, wässrigen Flüssigformulierung bildet einen weiteren Gegenstand der vorliegenden Erfindung. Das Verfahren ist dadurch gekennzeichnet, dass man den Farbbildner (a) mit dem anionischen Tensid (b) in Gegenwart von Wasser vermahlt und anschliessend das Verdickungsmittel (c) hinzugibt.

Das Mahlen wird in einem beliebigen Mühlentyp, beispielsweise einer Glaskugelmühle und vorzugsweise zwischen 10 und 60°C, vorzugsweise 15 und 20°C durchgeführt. Die Mischung aus dem Farbbildner (a) und dem anionischen Tensides (b) wird so lange gemahlen, bis eine Teilchengrösse von etwa 1 bis 2 µm erreicht ist. Erst dann wird das Verdickungsmittel hinzugerührt. Gegebenenfalls können die erfindungsgemässen Zusammensetzungen Antimkrobika enthalten. Weitere Zusätze, wie beispielsweise Antischaummittel sind für die erfindungsgemässen Flüssigformulierungen nicht erforderlich.

Die erfindungsgemässen Flüssigformulierungen stellen hochkonzentrierte wässrige Mischungen dar, die sich durch einfache Handhabung und eine ausgezeichnete Lagerstabilität auszeichnen.

Die erfindungsgemässen Flüssigformulierungen finden Verwendung in wärmempfindlichen Aufzeichnungsmaterialien.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Datenverarbeitungsmaschinen, Druckern, Faksimile- oder Kopiermaschinen oder in medizinischen und technischen Aufzeichnungsgeräten und Messinstrumenten verwendet. Die Bilderzeugung (Markierung) kann auch manuell mit einem beheizten Griffel oder einer beheizten Feder erfolgen. Eine weitere Einrichtung zur Erzeugung von Markierungen mittels Wärme sind Laserstrahlen.

Die erfindungsgemässe Flüssigformulierung wird zur Herstellung eines Überzugsgemisches mit einem Koreaktanden innig verrührt. Als Koreaktanden kommen organische Verbindungen mit einer phenolischen Hydroxygruppe in Betracht. Diese können sowohl einwertige als auch mehrwertige Phenole sein. Diese Phenole können durch Halogenatome, Carboxylgruppen, Alkylreste, Acylreste, wie Arylsulfonyl, oder Alkoxycarbonylreste, wie Benzyloxycarbonyl substituiert sein.

Spezielle Beispiele für geeignete Phenole sind 4-tert.-Butylphenol, Methylen-bis-(p-phenylphenol), 4-Hydroxydiphenylether, α-Naphthol, β-Naphthol, 4-Hydroxybenzoesäuremethylester oder -benzylester, 2-4-Dihydroxybenzoesäuremethylester, 4-Hydroxydiphenylsulfon, 4'-Hydroxy-4-methyldiphenylsulfon, 4'-Hydroxy-4-isopropoxydiphenylsulfon, 4-Hydroxy-acetophenon, 2,4-Dihydroxybenzophenon, 2,2'-Dihydroxydiphenyl, 2,4-Dihydroxydiphenylsulfon, 4,4'-Cyclohexylidendiphenol, 4,4'-Isopropylidendiphenol, 4,4'-Isopropyliden-bis-(2-methylphenol), 4,4-Bis-(4-hydroxyphenyl)valeriansäure, Resorcin, Hydrochinon, Pyrogallol, Phloroglucin, p-, m-, o-Hydroxybenzoesäure, 3,5-Di-(α-methylbenzyl)-salicylsäure, 3,5-Di-(α,α-dimethylbenzyl)-salicylsäure, Salicylosalicylsäure, Hydroxyphthalsäure, 1-Hydroxy-2-naphthoesäure oder Phenol-Formaldehyd-Vorpolymerisate, die auch mit Zink modifiziert sein können. Von den aufgezählten Carbonsäuren sind die Salicylsäurederivate bevorzugt, die vorzugsweise als Zinksalze eingesetzt werden. Besonders bevorzugte Zinksalicylate sind in der EP-A-181,283 oder DE-A-2,242,250 beschrieben. Besonders bevorzugte Koreaktanden sind Zinksalicylate, metallfreie Phenole, Phenolharze oder zinkmodifizierte Phenolharze.

Mittels Pumpen, Dosiervorrichtungen und Auftragsystemen kann die Zusammensetzung dem betreffenden Überzugsgemisch in genau abgemessenen Mengen zugesetzt werden.

Das Überzugsgemisch enthaltend die erfindungsgemässe Formulierung und den Koreaktanden wird mit einem geeigneten Auftragsystem, wie z.B. einer Rakel auf das betreffende thermoreaktive Aufzeichnungsmaterial als dünne Schicht aufgebracht. Das Auftragsgewicht liegt zwischen 0,5 bis 1 g/m². Die Schicht wird in spezifischen Bezirken mittels Wärme erweicht oder verschmolzen, wobei an den Punkten, an denen Wärme angewandt wird, sich sofort die gewünschte Farbe entwickelt.

Die thermoreaktiven Schichten können weitere Zusätze enthalten. Zur Verbesserung des Weissgrades oder der Thermokopfeignung des Aufzeichnungsmaterials und zur Verhinderung des Festklebens der erhitzten Feder oder Platte können diese Schichten z.B. Antioxidantien, UV-Absorber, Lösungshilfen, Talk, Titandioxid, Zinkoxid, Aluminiumoxid, Aluminiumhydroxid, Calciumcarbonat (z.B. Kreide), Tone oder auch organische Pigmente, wie z.B. Harnstoff-Formaldhydpolymerisate, enthalten. Um zu verhindern, dass die Farbe nur innerhalb eines begrenzten Temperaturbereiches gebildet wird, können Substanzen, wie Harnstoff, Thioharnstoff, Diphenylthioharnstoff Acetamid, Acetanilid, Benzosulfanilid, Bis-stearoylethylendiamid, Stearinsäureamid, Phthalsäureanhydrid, Benzoyloxybenzoesäurebenzylester, Metallstearate wie z.B. Zinkstearat, Phthalsäurenitril, Dimethylterephthalat, Dibenzyltherephthalat, Dibenzylisophthalat, Benzyldiphenyl oder andere schmelzbare Produkte, welche das gleichzeitige Schmelzen der Farbbildner und des Koreaktanden induzieren, zugesetzt werden. Bevorzugt enthalten thermographische Aufzeichnungsmaterialien Wachse, z.B. Carnaubawachse, Montanwachse, Paraffinwachse, Polyethylenwachse, Kondensate höherer Fettsäureamide und Formaldehyd oder Kondensate höherer Fettsäuren und Ethylendiamin.

In den folgenden Herstellungs- und Applikationsbeispielen beziehen sich die angegebenen Prozentsätze, wenn nicht anders angegeben ist, auf das Gewicht. Teile sind Gewichtsteile.

### Herstellungsbeispiele

### Beispiel 1: Zu 10 g (40%) des Farbbildners der Formel

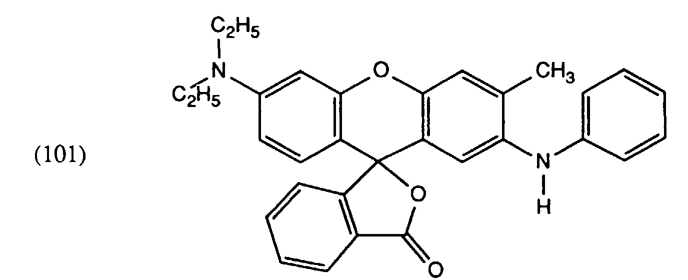

wird 1,25 g (5%) des Natriumsalzes des Sulfobernsteinsäuremonoesters auf Basis eines ethoxylierten Alkanolamids und 13,5 g (54%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,25 g des Verdickungsmittels Gummi Arabicum zugegeben.

Beispiel 2: Zu 10 g (40%) des Farbbildners der Formel (101) wird 1,25 g (5%) des sulfonierten Monoesters einer C₁₂-C₂₂Fettsäure und 13,5 g (54%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,25 g (1%) des Verdickungsmittels Xanthan Gum zugegeben.

Beispiel 3: Zu 10 g (40%) des Farbbildners der Formel (101) wird 1,25 g (5%) des sulfonierten Monoesters einer C₁₂-C₂₂Fettsäure und 13,5 g (54%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,25% des Verdickungsmittels Polyvinylalkohol 100,000 zugegeben.

Beispiel 4: Zu 10 g (40%) des Farbbildners der Formel (101) wird 1,25 g (5%) des sulfonierten Monoesters einer C₁₂-C₂₂Fettsäure und 13,5 g (54%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,25 g (1%) des Verdickungsmittels Natriumalginat zugegeben.

Beispiel 5: Zu 10 g (40%) des Farbbildners der Formel (101) wird 1,25 g (5%) des sulfonierten Monoesters einer C₁₂-C₂₂Fettsäure und 13,625 g (54,5%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,125% des Verdickungsmittels CMC zugegeben.

Beispiel 6: Zu 10 g (40%) des Farbbildners der Formel (101) wird 1,25 g (5%) des sulfonierten Monoesters einer C₁₂-C₂₂Fettsäure und 13,4625 g (53,85%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,25 g (1%) der Verdickungsmittel NTC 80 und 0,0375 g (0,15%) Xanthan Gum zugegeben.

Beispiel 7: Zu 10 g (40%) des Farbbildners der Formel (101) wird 1,25 g (5%) des sulfonierten Monoesters einer C₁₂-C₂₂Fettsäure und 13,625 g (54,5%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,125 g (0,5%) des Verdickungsmittels κ-Karageen zugegeben.

Beispiel 8: Zu 10 g (40%) des Farbbildners der Formel (101) wird 1,25 g (5%) des sulfonierten Monoesters einer C₁₂-C₂₂Fettsäure und 13,5 g (54%) Wasser gegeben. Zusätzlich werden 50 g Glaskugeln (Durchmesser 1 mm) zugegeben. Die Mischung wird so lange gemahlen, bis die Teilchengrösse kleiner als 2 µ vorzugsweise 1-1,5 µ ist. Die Glaskugeln werden abgetrennt. Anschliessend wird der Mischung 0,5 g (2%) des Verdickungsmittels Polyacrylsäure 170,000 Natriumsalz zugegeben.

Beispiel 9: Zu 10 g (40%) des Farbbildners der Formel (101) wird 1,25 g (5%) des sulfonierten Monoesters einer C₁₂-C₂₂Fettsäure und 13,5 g (54%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,25 g (1%) des Verdickungsmittels Bentonit zugegeben.

Beispiel 10: Zu 10 g (40%) des Farbbildners der Formel (101) wird 1,25 g (5%) des Natriumsalzes des Sulfobernsteinsäuremonoesters auf Basis eines ethoxylierten Alkanolamids und 13,69 g (54,75%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,063 g (0,25%) eines Ethylenoxid-Propylenoxid-Copolymers mit einem Molekulargewicht von ca. 2000 als Verdickungsmittel zugegeben.

Beispiel 11: Zu 10 g (40%) des Farbbildners der Formel (101) wird 1,25 g (5%) des Natriumsalzes des Sulfobernsteinsäuremonoesters auf Basis eines ethoxylierten Alkanolamids und 12,5 g (50%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Danach wird der Mischung 1,25 g des Anlagerungsproduktes aus einem Mol eines C₁₃-Oxoalkohols und 3 Mol Ethylenoxid als Verdickungsmittel zugegeben.

Beispiel 12: Zu 10 g (40%) des Farbbildners der Formel (101) wird 1,25 g (5%) des anionischen Tensids der Formel 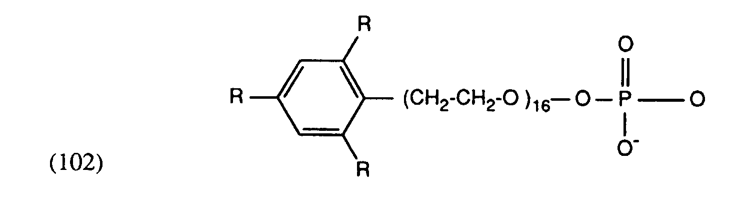 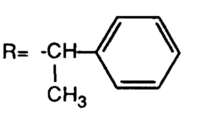 und 12,5 g (50%) Wasser gegeben. Zusätzlich werden 50 g Glaskugeln (Durchmesser 1 mm) zugegeben. Die Mischung wird so lange gemahlen, bis die Teilchengrösse kleiner als 2 µ, vorzugsweise 1-1,5 µ ist. Die Glaskugeln werden abgetrennt. Anschliessend wird der Mischung 0,25 g (1%) des Verdickungsmittels Natriumalginat zugegeben.

Beispiel 13: Man verfährt wie in Beispiel 12 angegeben mit dem Unterschied, dass man anstelle von 0,25 g des Verdickungsmittels Natriumalginat 0,25 g (1 %) des Verdickungsmittels Bentonit zugibt.

Beispiel 14: Anstelle des Farbbildners der Formel (101), der in den Beispielen 1 bis 13 eingesetzt wird, kann man auch den Farbbildner der Formel 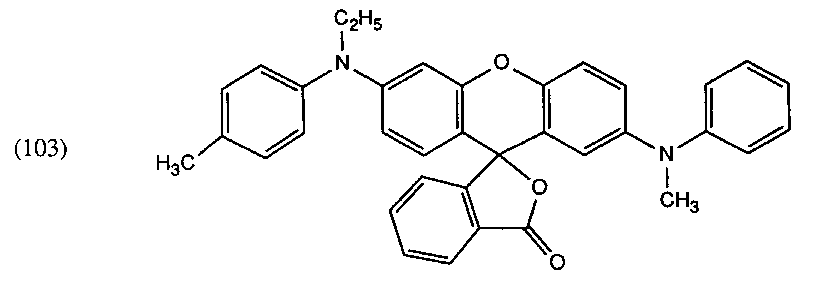 verwenden.

Beispiel 15: Anstelle des Farbbildners der Formel (101), der in den Beispielen 1 bis 13 eingesetzt wird, kann man auch den Farbbildner der Formel 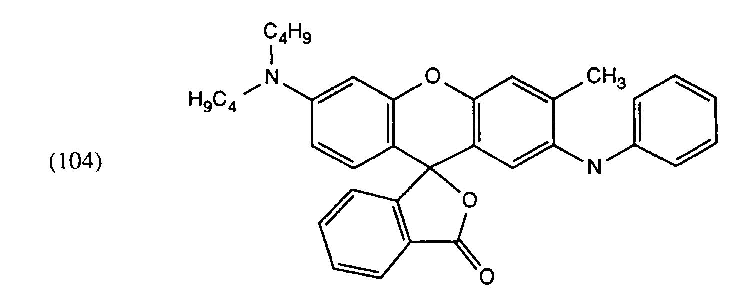 verwenden.

### Applikationsbeispiele

Beispiel 16: 0,6 g der nach Beispiel 1 hergestellten Flüssigformulierung wird mit 13,38 g einer Koreaktandmischung enthaltend
3,8% Bisphenyl und
3,0 % Benzyldiphenyl
vermischt und mit einem Rakel so auf ein Papier aufgetragen, dass das Flächengewicht 0,5 g/m² entspricht. Durch Verwendung des Papiers in einem Faksimile-Gerät (Infotec der Firma CANON) entwickelt sich eine lichtechte, intensiv schwarze Farbe.

Beispiele 17 - 23: Verwendet man anstelle der nach Beispiel 1 hergestellten Flüssigformulierung die nach den Beispielen 2-15 verwendeten Flüssigformulierungen und verfährt im übrigen wie in Beispiel 16 angegeben, so erhält man ebenfalls lichtechte, intensiv schwarze Farben.

## Patentansprüche

1. Konzentrierte Flüssigformulierungen von Farbbildnern enthaltend
a) als Farbbildner eine Fluoranverbindung der Formel 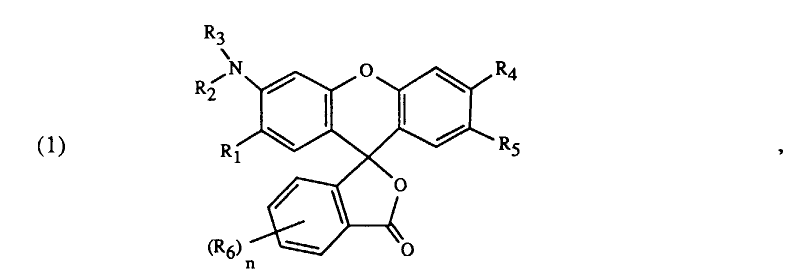 worin
R₁ Wasserstoff oder C₁-C₄-Alkyl;
R₂ und R₃ unabhängig voneinander Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄Alkyl; 2-Tetrahydrofuranyl, oder
R₂ und R₃ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
R₄ Wasserstoff, Hydroxy oder C₁-C₄-Alkyl;
R₅ Nitro; SO₂R₇; SO₂OR₈; SO₂NR₉R₁₀; COR₁₁; CONR₉R₁₀; oder C₁-C₄-Halogenalkyl; nicht substituierter oder durch Halogen, C₁-C₄-Alkyl, C₁-C₄-Halogenalkyl oder C₁-C₄-Alkoxy substituiertes C₆-C₁₀-Aryl; nicht substituierter oder durch Halogen oder Hydroxy substituierter 2-Triazinyl- oder 1-Benztriazolylrest;
R₆ Halogen; Nitro; C₁-C₄-Alkyl; C₁-C₄-Halogenalkyl; Amino; mono-C₁-C₄-Alkylamino; di-C₁-C₄-Alkylamino; oder COR₁₁;
n 0; 1; 2; 3; oder 4;
R₇ C₁-C₈-Alkyl; oder C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-Alkyl;
R₈ Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
R₉ und R₁₀ unabhängig voneinander Wasserstoff; oder C₁-C₈-Alkyl; oder
R₉ und R₁₀ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring; und
R₁₁ Wasserstoff; Hydroxy; C₁-C₈-Alkyl; C₁-C₈-Alkoxy; C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy
bedeutet,
(b) als anionische Tenside
(ba) sulfatierte Alkane
(bb) Alkansulfonate
(bc) sulfonierte Carboxylate oder
(bd) sulfatierte Carboxylate, oder
(be) sauere Ester oder deren Salze von Alkylenoxidaddukten und
(c) ein Verdickungsmittel.

2. Flüssigformulierung nach Anspruch 1, dadurch gekennzeichnet, dass als Farbbildner eine Fluoranverbindung der Formel (1) verwendet wird, worin
R₁ Wasserstoff oder C₁-C₄-Alkyl;
R₂ und R₃ unabhängig voneinander Wasserstoff; C₁-C₅-Alkyl; oder
R₂ und R₃ gemeinsam mit dem Stickstoffatom, an welches sie gebunden sind, einen unsubstituierten oder durch C₁-C₄-Alkyl substituierten Pyrrolidin- oder Piperidin-Ring;
R₄ Wasserstoff oder C₁-C₄-Alkyl;
R₅ Nitro; SO₂R₇; SO₂OR₈; SO₂NR₉R₁₀; COR₁₁; CONR₉R₁₀; C₁-C₄-Halogenalkyl; unsubstituiertes oder im Phenylrest durch C₁-C₄-Alkyl; C₁-C₄-Halogenalkyl, C₁-C₄-Alkoxy substituiertes Phenyl; Phenylamino; oder Phenyl-C₁-C₄-Alkylamino;
n 0; 1; 2; 3; oder 4;
R₆ wenn n 1, 2, 3 oder 4 ist; Halogen; wenn n 1 oder 2 ist C₁-C₄-Alkyl; C₁-C₄-Halogenalkyl; oder wenn n 1 ist, Nitro, COR₁₁; Amino, mono-C₁-C₄-Alkylamino oder di-C₁-C₄-Alkylamino;
R₇ C₁-C₄-Alkyl; oder C₁-C₄-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₂-Alkyl;
R₈ Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₂-alkyl;
R₉ und R₁₀ unabhängig voneinander Wasserstoff; oder C₁-C₈-Alkyl; oder
R₉ und R₁₀ gemeinsam mit dem Stickstoffatom, an welches sie gebunden sind, einen unsubstituierten oder durch C₁-C₄-Alkyl substituierten Pyrrolidin- oder Piperidin-Ring; und
R₁₁ Wasserstoff; C₁-C₄-Alkyl; C₁-C₄-Halogenalkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy
bedeutet.

3. Flüssigformulierung nach Anspruch 1 oder 2, dadurch gekennzeichnet, dass als Farbbildner eine Fluoranverbindung der Formel (1) verwendet wird, worin
R₁ Wasserstoff oder Methyl;
R₂ und R₃ unabhängig voneinander Wasserstoff; C₁-C₅-Alkyl; oder
R₂ und R₃ gemeinsam mit dem Stickstoffatom, an welches sie gebunden sind, einen unsubstituierten Pyrrolidin- oder Piperidin-Ring;
R₄ Wasserstoff oder Methyl;
R₅ Nitro; SO₂R₇; SO₂NR₉R₁₀; COR₁₁; CONR₉R₁₀; C₁-C₄-Halogenalkyl; unsubstituiertes oder im Phenylrest durch Halogen, C₁-C₄-Alkyl; C₁-C₄-Halogenalkyl oder C₁-C₄-Alkoxy substituiertes Phenyl; Phenyl-C₁-C₄-Alkyl; Phenylamino; oder Phenyl-C₁-C₄-Alkylamino;
n 0; 1; 2; 3; oder 4;
R₆ wenn n 1, 2, 3 oder 4 ist; Halogen; wenn n 1 oder 2 ist Methyl; oder wenn n 1 ist, Nitro, Amino, mono-C₁-C₄-Alkylamino oder di-C₁-C₄-Alkylamino;
R₇ C₁-C₄-Alkyl; oder C₁-C₄-Halogenalkyl; unsubstituiertes oder im Phenylrest durch Halogen, C₁-C₄-Alkyl oder C₁-C₄-Alkoxy substituiertes Phenyl;
R₉ und R₁₀ unabhängig voneinander Wasserstoff; oder C₁-C₄-Alkyl;
R₁₁ Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy
bedeutet.

4. Flüssigformulierungen nach einem der Ansprüche 1 bis 3 enthaltend mindestens einen Farbbildner der Formel (1) worin
R₅ COR₁₁ bedeutet.

5. Flüssigformulierungen nach Anspruch 4, dadurch gekennzeichnet, dass
R₁₁ C₁-C₄-Alkyl, C₁-C₄-Alkoxy oder Phenyl-C₁-C₂-Alkoxy bedeutet.

6. Flüssigformulierungen nach einem der Ansprüche 1 bis 3 enthaltend mindestens einen Farbbildner der Formel (1) worin
R₅ Phenyl; Phenylamino; oder Phenyl-C₁-C₄-Alkylamino bedeutet.

7. Flüssigformulierungen nach Anspruch 1, dadurch gekennzeichnet, dass als Farbbildner eine Fluoranverbindung der Formel (1) verwendet wird, worin
R₂ und R₃ unabhängig voneinander Wasserstoff; C₁-C₁₂-Alkyl, C₅-C₇-Cycloalkyl, C₃-C₅-Alkenyl oder Benzyl bedeuten, oder
R₂ und R₃ gemeinsam mit dem Stickstoffatom, an welches sie gebunden sind, einen heterocyclischen Rest bilden.

8. Flüssigformulierungen nach Anspruch 7, dadurch gekennzeichnet, dass als Farbbildner eine Fluoranverbindung der Formel (1) verwendet wird, worin
R₂ und R₃ unabhängig voneinander C₁-C₄-Alkyl bedeuten.

9. Flüssigformulierungen nach einem der Ansprüche 1 bis 8, dadurch gekennzeichnet, dass man als anionische Tenside (ba)
Salze der Schwefelsäureester von Fettalkoholen der Formel
(2) R₁₂-O-SO₃-M₁,
worin R₁₂ einen Alkylrest von 6 bis 18 Kohlenstoffatomen und
M₁ Alkalimetall
bedeutet, verwendet.

10. Flüssigformulierungen nach einem der Ansprüche 1 bis 8, dadurch gekennzeichnet, dass man als anionische Tenside (bb) sekundäre Alkansulfonate mit einer Alkylkettenlänge von 14 bis 18 Kohlenstoffatomen verwendet.

11. Flüssigformulierungen nach einem der Ansprüche 1 bis 8, dadurch gekennzeichnet, dass man als anionische Tenside (bc) Verbindungen der Formel 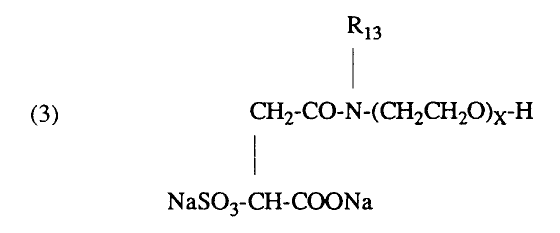 worin
R₁₃ Wasserstoff oder (CH₂CH₂O)ₓH und
x 1 bis 10 bedeutet, verwendet.

12. Flüssigformulierungen nach einem der Ansprüche 1 bis 8, dadurch gekennzeichnet, dass man
als anionisches Tensid (bd) Alkalimetall-, Ammonium- oder Aminsalze der Schwefelsäureester von Fettsäuren mit 10 bis 22 Kohlenstoffatomen, verwendet.

13. Flüssigformulierungen nach einem der Ansprüche 1 bis 8, dadurch gekennzeichnet, dass man als anionische Tenside (be) eine Verbindung der Formel 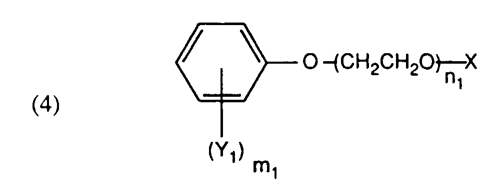 verwendet,
worin
Y₁ C₄-C₁₂-Alkyl, Phenyl, Tolyl, Phenyl-C₁-C₃-Alkyl oder Tolyl-C₁-C₃-Alkyl bedeutet,
X₁ ein Säurerest ist, der sich von Schwefelsäure oder Orthophosphorsäure ableitet, und
m₁ 1 bis 3 und n₁ 4 bis 40 sind.

14. Flüssigformulierungen nach Anspruch 13, dadurch gekennzeichnet, dass man als anionisches Tensid (be) eine Verbindung der Formel 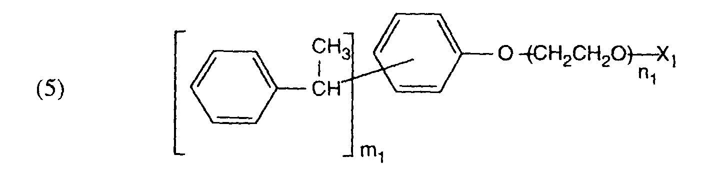 verwendet, worin
X₁ ein Säurerest ist, der sich von Schwefelsäure oder Orthophosphorsäure ableitet, und
m₁ 1 bis 3 und
n₁ 4 bis 40 sind.

15. Flüssigformulierungen nach einem der Ansprüche 1 bis 14, dadurch gekennzeichnet, dass man als Verdickungsmittel
(ca) ionisch modifizierte Polysaccharide,
(cb) nichtionische Cellulose und deren Derivate,
(cc) synthetische, wasserlösliche Carboxylate,
(cd) Polyvinylalkohol oder
(ce) Bentonite und
(cf) nichtionogene Tenside
verwendet.

16. Flüssigformulierungen nach Anspruch 15, dadurch gekennzeichnet, dass man als Verdickungsmittel ionisch modifizierte Polysaccharide verwendet.

17. Flüssigformulierungen nach Anspruch 15 oder 16, dadurch gekennzeichnet, dass man als Verdickungsmittel Natriumalginat verwendet.

18. Flüssigformulierungen nach Anspruch 15 oder 16, dadurch gekennzeichnet, dass man als Verdickungsmittel Xanthan verwendet.

19. Flüssigformulierungen nach Anspruch 15, dadurch gekennzeichnet, dass man als Komponente (cc) Polyacrylsäure mit einem Molekulargewicht von 150,000 bis 200,000 verwendet.

20. Flüssigformulierungen nach Anspruch 15, dadurch gekennzeichnet, dass man als Verdickungsmittel Polyvinylalkohol mit einem Molekulargewicht von 15,000 bis 1,500,000 verwendet.

21. Flüssigformulierungen nach Anspruch 15, dadurch gekennzeichnet, dass man als nichtionogenes Tensid (cf) ein Copolymer aus Ethylenoxid und Propylenoxid verwendet.

22. Flüssigformulierungen nach Anspruch 15, dadurch gekennzeichnet, dass man als nichtionogenes Tensid (cf) ein Alkylenoxiaddukt an gesättigte oder ungesättigte 1-6-wertige aliphatische Alkohole verwendet.

23. Flüssigformulierungen nach Anspruch 1, dadurch gekennzeichnet, dass sie
(a) einen Farbbildner der Formel (1),
(b) ein Alkalimetallsalz des Schwefelsäureesters einer C₁₀-C₁₈Fettsäure und
(c) ein ionisch modifiziertes Polysaccharid enthaltne.

24. Flüssigformulierungen nach Anspruch 23, dadurch gekennzeichnet, dass sie
(a) einen Farbbildner der Formel (1), worin
R₂ und R₃ unabhängig voneinander C₁-C₄Alkyl und
R₅ Phenyl; Phenylamino; oder Phenyl-C₁-C₄-Alkylamino bedeuten,
(b) das Natriumsalz des Schwefelsäureesters einer C₁₀-C₁₈Fettsäure und
(c) Natriumalginat
enthalten.

25. Flüssigformulierungen nach Anspruch 23, dadurch gekennzeichnet, dass sie
(a) einen Farbbildner der Formel (1), worin
R₂ und R₃ unabhängig voneinander C₁-C₄Alkyl und
R₅ Phenyl; Phenylamino; oder Phenyl-C₁-C₄-Alkylamino bedeuten,
(b) ein anionisches Tensid der Formel (3) und
(c) Natriumalginat enthalten.

26. Flüssigformulierungen nach Anspruch 1, dadurch gekennzeichnet, dass sie
(a) einen Farbbildner der Formel (1),
(b) ein Alkalimetallsalz des Schwefelsäureesters einer C₁₀-C₁₈Fettsäure und
(c) ein in Wasser schwerlösliches nichtionogenes Tensid enthalten.

27. Flüssigformulierungen nach einem der Ansprüche 1 bis 26, dadurch gekennzeichnet, dass sie
20 bis 60 Gew.% eines Farbbildners (a)
1 bis 15 Gew.% eines anionischen Tensides (b),
0,1 bis 5 Gew.% eines Verdickungsmittels (c) und
ad 100 % Wasser
enthalten.

28. Verfahren zur Herstellung der Flüssigformulierungen nach einem der Ansprüche 1 bis 27 dadurch gekennzeichnet, dass man den Farbbildner (a) mit dem anionischen Tensid (b) in Gegenwart von Wasser vermahlt und anschliessend das Verdickungsmittel (c) hinzugibt.

29. Verwendung der Flüssigformulierungen nach einem der Ansprüche 1 bis 27 als wärmeempfindliches Aufzeichnungsmaterial.

## Claims

1. A concentrated liquid formulation of a colour former comprising
a) as colour former, a fluoran compound of formula 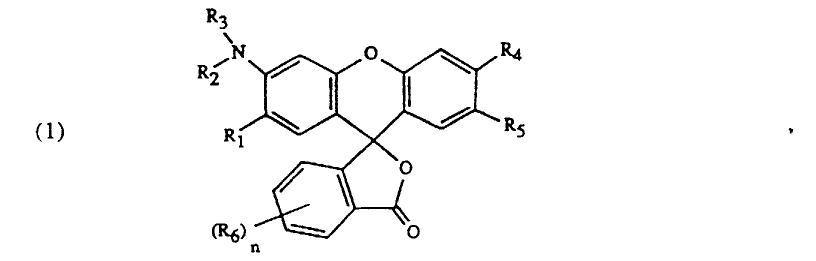 wherein
R₁ is hydrogen or C₁-C₄alkyl;
R₂ and R₃ are each independently of the other hydrogen; C₁-C₈alkyl; unsubstituted or C₁-C₄alkyl- or halogen-substituted C₄-C₇cycloalkyl; unsubstituted phenyl or phenyl which is substituted by C₁-C₄alkyl, hydroxy or halogen; phenyl-C₁-C₄alkyl; C₃-C₆alkenyl; C₁-C₄alkoxy; C₁-C₄alkoxy-C₁-C₄alkyl; 2-tetrahydrofuranyl, or
R₂ and R₃, together with the linking nitrogen atom, are an unsubstituted or C₁-C₄alkyl-substituted pyrrolidine, piperidine, morpholine, thiomorpholine or piperazine ring;
R₄ is hydrogen, hydroxy or C₁-C₄alkyl;
R₅ is nitro; SO₂R₇; SO₂OR₈; SO₂NR₉R₁₀; COR₁₁; CONR₉R₁₀; or C₁-C₄haloalkyl; C₆-C₁₀aryl which is unsubstituted or substituted by halogen, C₁-C₄alkyl, C₁-C₄haloalkyl or C₁-C₄alkoxy; an unsubstituted or a halogen- or hydroxy-substituted 2-triazinyl or 1-benzotriazolyl radical;
R₆ is halogen; nitro; C₁-C₄alkyl; C₁-C₄haloalkyl; amino; mono-C₁-C₄alkylamino; di-C₁-C₄alkylamino; or COR₁₁;
n is 0; 1; 2; 3; or 4;
R₇ is C₁-C₈alkyl; or C₁-C₈haloalkyl; unsubstituted phenyl or phenyl-C₁-C₄alkyl, or phenyl or phenyl-C₁-C₄alkyl which is substituted in the phenyl radical by halogen, C₁-C₄alkyl, C₁-C₄haloalkyl, C₁-C₄alkoxy;
R₈ is hydrogen; C₁-C₈alkyl; C₁-C₈haloalkyl; unsubstituted phenyl or phenyl-C₁-C₄alkyl, or phenyl or phenyl-C₁-C₄alkyl which is substituted in the phenyl radical by halogen, C₁-C₄alkyl, C₁-C₄haloalkyl, C₁-C₄alkoxy;
R₉ and R₁₀ are each independently of the other hydrogen; or C₁-C₈alkyl; or
R₉ and R₁₀, together with the linking nitrogen atom, are an unsubstituted or C₁-C₄alkyl-substituted pyrrolidine, piperidine, morpholine, thiomorpholine or piperazine ring; and
R₁₁ is hydrogen; hydroxy; C₁-C₈alkyl; C₁-C₈alkoxy; C₁-C₈-haloalkyl; unsubstituted phenyl or phenyl which is substituted by halogen, C₁-C₄alkyl, C₁-C₄haloalkyl, C₁-C₄alkoxy; phenyl-C₁-C₄-alkyl or phenyl-C₁-C₄alkoxy,
(b) as anionic surfactants,
(ba) sulfated alkanes
(bb) alkanesulfonates
(bc) sulfonated carboxylates or
(bd) sulfated carboxylates, or
(be) acidic esters or salts thereof of alkylene oxide adducts, and
(c) a thickener.

2. A liquid formulation according to claim 1, wherein the colour former used is a fluoran compound of formula (1), wherein
R₁ is hydrogen or C₁-C₄alkyl;
R₂ and R₃ are each independently of the other hydrogen; C₁-C₅alkyl; or
R₂ and R₃, together with the linking nitrogen atom, are an unsubstituted or C₁-C₄alkyl-substituted pyrrolidine or piperidine ring;
R₄ is hydrogen or C₁-C₄alkyl;
R₅ is nitro; SO₂R₇; SO₂OR₈; SO₂NR₉R₁₀; COR₁₁; CONR₉R₁₀; C₁-C₄haloalkyl; unsubstituted phenyl or phenyl which is substituted by C₁-C₄alkyl, C₁-C₄haloalkyl, C₁-C₄alkoxy; phenylamino; or phenyl-C₁-C₄alkylamino;
n is 0; 1; 2; 3; or 4;
R₆, if n is 1, 2, 3 or 4, is halogen; if n is 1 or 2, is C₁-C₄alkyl, C₁-C₄haloalkyl; or, if n is 1, is nitro, COR₁₁, amino, mono-C₁-C₄alkylamino or di-C₁-C₄alkylamino;
R₇ is C₁-C₄alkyl; or C₁-C₄haloalkyl; unsubstituted phenyl or phenyl-C₁-C₂alkyl, or phenyl or phenyl-C₁-C₂-alkyl which is substituted in the phenyl radical by halogen, C₁-C₄alkyl, C₁-C₄haloalkyl or C₁-C₄alkoxy;
R₈ is hydrogen; C₁-C₄alkyl; C₁-C₄haloalkyl; unsubstituted phenyl or phenyl-C₁-C₂alkyl, or phenyl or phenyl-C₁-C₂alkyl which is substituted in the phenyl radical by halogen, C₁-C₄alkyl, C₁-C₄haloalkyl or C₁-C₄alkoxy;
R₉ and R₁₀ are each independently of the other hydrogen; or C₁-C₈alkyl; or
R₉ and R₁₀, together with the linking nitrogen atom, are an unsubstituted or C₁-C₄alkyl-substituted pyrrolidine or piperidine ring; and
R₁₁ is hydrogen; C₁-C₄alkyl; C₁-C₄haloalkyl; C₁-C₄alkoxy; unsubstituted phenyl or phenyl which is substituted by halogen, C₁-C₄alkyl, C₁-C₄haloalkyl or C₁-C₄alkoxy; phenyl-C₁-C₂-alkyl or phenyl-C₁-C₂alkoxy.

3. A liquid formulation according to claim 1 or 2, wherein the colour former used is a fluoran compound of formula (1), wherein
R₁ is hydrogen or methyl;
R₂ and R₃ are each independently of the other hydrogen; C₁-C₅alkyl; or
R₂ and R₃, together with the linking nitrogen atom, are an unsubstituted pyrrolidine or piperidine ring;
R₄ is hydrogen or methyl;
R₅ is nitro; SO₂R₇; SO₂NR₉R₁₀; COR₁₁; CONR₉R₁₀; C₁-C₄haloalkyl; unsubstituted phenyl or phenyl which is substituted by halogen, C₁-C₄alkyl, C₁-C₄haloalkyl or C₁-C₄alkoxy; phenyl C₁-C₄alkyl; phenylamino; or phenyl-C₁-C₄alkylamino;
n is 0; 1; 2; 3; or 4;
R₆, if n is 1, 2, 3 or 4, is halogen; if n is 1 or 2, is methyl; or, if n is 1, is nitro, amino, mono-C₁-C₄₋alkylamino or di-C₁-C₄alkylamino;
R₇ is C₁-C₄alkyl; or C₁-C₄haloalkyl; unsubstituted phenyl or phenyl which is substituted by halogen, C₁-C₄alkyl or C₁-C₄alkoxy;
R₉ and R₁₀ are each independently of the other hydrogen; or C₁-C₄alkyl;
R₁₁ is hydrogen; C₁-C₄alkyl; C₁-C₄alkoxy; unsubstituted phenyl or phenyl which is substituted by halogen, C₁-C₄alkyl, C₁-C₄haloalkyl or C₁-C₄alkoxy; phenyl-C₁-C₂-alkyl or phenyl-C₁-C₂alkoxy.

4. A liquid formulation according to any one of claims 1 to 3, comprising at least one colour former of formula (1), wherein
R₅ is COR₁₁.

5. A liquid formulation according to claim 4, wherein R₁₁ is C₁-C₄alkyl, C₁-C₄alkoxy or phenyl-C₁-C₂alkoxy.

6. A liquid formulation according to any one of claims 1 to 3, comprising at least one colour former of formula (1), wherein
R₅ is phenyl; phenylamino; or phenyl-C₁-C₄alkylamino.

7. A liquid formulation according to claim 1, wherein the colour former used is a fluoran compound of formula (1), wherein
R₂ and R₃ are each independently of the other hydrogen; C₁-C₁₂alkyl, C₅-C₇cycloalkyl, C₃-C₅alkenyl or benzyl, or R₂ and R₃, together with the linking nitrogen atom, form a heterocyclic radical.

8. A liquid formulation according to claim 7, wherein the colour former used is a fluoran compound of formula (1), wherein
R₂ and R₃ are each independently of the other C₁-C₄alkyl.

9. A liquid formulation according to any one of claims 1 to 8, wherein the anionic surfactant (ba) used is a salt of sulfuric esters of fatty alcohols of formula
(2) R₁₂-O-SO₃-M₁,
wherein R₁₂ is an alkyl radical of 6 to 18 carbon atoms, and
M₁ is alkali metal.

10. A liquid formulation according to any one of claims 1 to 8, wherein the anionic surfactant (bb) used is a secondary alkanesulfonate having an alkyl chain length of 14 to 18 carbon atoms.

11. A liquid formulation according to any one of claims 1 to 8, wherein the anionic surfactant (bc) used is a compound of formula 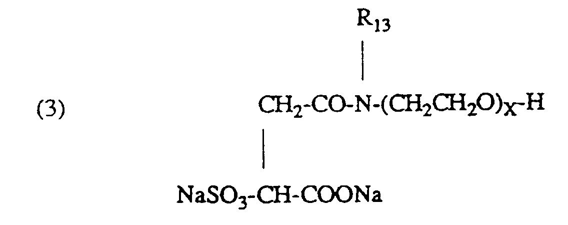 wherein
R₁₃ is hydrogen or (CH₂CH₂O)ₓH and
x is 1 to 10.

12. A liquid formulation according to any one of claims 1 to 8, wherein
the anionic surfactant (bd) used is an alkali metal salt, ammonium salt or amine salt of sulfuric esters of fatty acids having 10 to 22 carbon atoms.

13. A liquid formulation according to any one of claims 1 to 8, wherein the anionic surfactant (be) used is a compound of formula 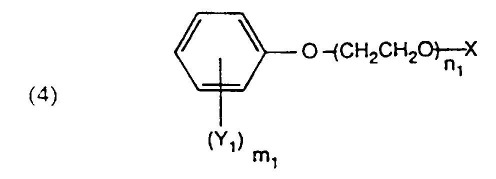 wherein
Y₁ is C₄-C₁₂alkyl, phenyl, tolyl, phenyl-C₁-C₃alkyl or tolyl-C₁-C₃alkyl,
X₁ is an acid radical derived from sulfuric acid or orthophosphoric acid, and
m₁ is 1 to 3 and n₁ is 4 to 40.

14. A liquid formulation according to claim 13, wherein the anionic surfactant (be) used is a compound of formula 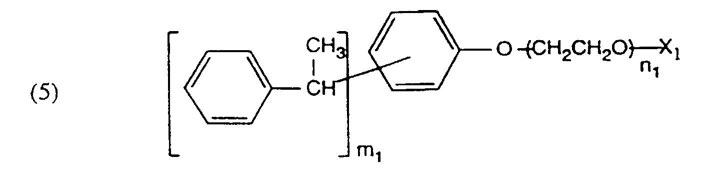 wherein
X₁ is an acid radical derived from sulfuric acid or orthophosphoric acid, and
m₁ is 1 to 3 and
n₁ is 4 to 40.

15. A liquid formulation according to any one of claims 1 to 14, wherein the thickener used is an
(ca) ionically modified polysaccharide,
(cb) nonionic cellulose or derivative thereof,
(cc) synthetic, water-soluble carboxylate,
(cd) polyvinyl alcohol or
(ce) bentonite, and
(cf) nonanionic surfactant.

16. A liquid formulation according to claim 15, wherein the thickener used is an ionically modified polysaccharide.

17. A liquid formulation according to claim 15 or 16, wherein the thickener used is sodium alginate

18. A liquid formulation according to claim 15 or 16, wherein the thickener used is xanthan.

19. A liquid formulation according to claim 15, wherein component (cc) is polyacrylic acid having a molecular weight of 150 000 to 200 000.

20. A liquid formulation according to claim 15, wherein the thickener used is polyvinyl alcohol having a molecular weight of 15 000 to 1 500 000.

21. A liquid formulation according to claim 15, wherein the nonionic surfactant (cf) used is a copolymer of ethylene oxide and propylene oxide.

22. A liquid formulation according to claim 15, wherein the nonionic surfactant (cf) used is an alkylene oxide adduct with saturated or unsaturated monohydric to hexahydric aliphatic alcohols.

23. A liquid formulation according to claim 1, comprising
(a) a colour former of formula (1),
(b) an alkali metal salt of the sulfuric ester of a C₁₀-C₁₈ fatty acid, and
(c) an ionically modified polysaccharide.

24. A liquid formulation according to claim 23, comprising
(a) a colour former of formula (1), wherein
R₂ and R₃ are each independently of the other C₁-C₄alkyl, and
R₅ is phenyl; phenylamino; or phenyl-C₁-C₄alkylamino,
(b) the sodium salt of the sulfuric ester of a C₁₀-C₁₈ fatty acid, and
(c) sodium alginate.

25. A liquid formulation according to claim 23, comprising
(a) a colour former of formula (1), wherein
R₂ and R₃ are each independently of the other C₁-C₄alkyl, and
R₅ is phenyl; phenylamino; or phenyl-C₁-C₄alkylamino,
(b) an anionic surfactant of formula (3), and
(c) sodium alginate.

26. A liquid formulation according to claim 1, comprising
(a) a colour former of formula (1),
(b) an alkali metal salt of the sulfuric ester of a C₁₀-C₁₈ fatty acid, and
(c) a sparingly water-soluble nonionic surfactant.

27. A liquid formulation according to any one of claims 1 to 26, comprising
20 to 60% by weight of a colour former (a),
1 to 15% by weight of an anionic surfactant (b),
0.1 to 5% by weight of a thickener (c), and
water to make up 100%.

28. A process for the preparation of a liquid formulation according to any one of claims 1 to 27, which comprises milling the colour former (a) with the anionic surfactant (b) in the presence of water and then adding the thickener (c).

29. The use of a liquid formulation according to any one of claims 1 to 27 as heat-sensitive recording material.

## Revendications

1. Formulations liquides concentrées d'agents chromogènes contenant
a) comme agent chromogène un fluorane de formule 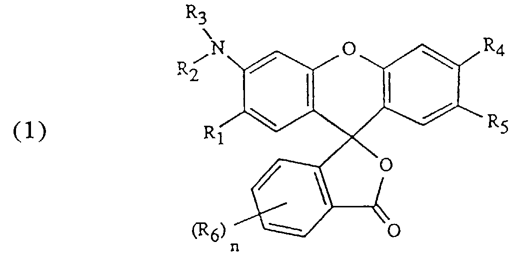 dans laquelle
R₁ représente un atome d'hydrogène ou un groupe alkyle en C₁₋₄,
R₂ et R₃ représentent, indépendamment l'un de l'autre un atome d'hydrogène; un groupe alkyle en C₁₋₈; un groupe cycloalkyle en C₄₋₇ non substitué ou portant un substituant alkyle en C₁₋₄ ou halogéno; un groupe phényle non substitué ou portant un substituant alkyle en C₁₋₄, hydroxy ou halogéno; un résidu phényl-(alkyle en C₁₋₄); alcényle en C₃₋₆; alcoxy en C₁₋₄, (alcoxy en C₁₋₄)-(alkyle en C₁₋₄); 2-tétrahydrofuranyle; ou
R₂ et R₃ représentent avec l'atome d'azote auquel ils sont liés, un noyau pyrrolidine, pipéridine, morpholine, thiomorpholine ou pipérazine non substitué ou portant un substituant alkyle en C₁₋₄;
R₄ représente un atome d'hydrogène ou un groupe hydroxy ou alkyle en C₁₋₄;
R₅ représente un groupe nitro; SO₂R₇; SO₂OR₈; SO₂NR₉R₁₀; COR₁₁; CONR₉R₁₀ ou halogénoalkyle en C₁₋₄; aryle en C₆₋₁₀ non substitué ou portant un substituant halogéno, alkyle en C₁₋₄, halogénoalkyle en C₁₋₄ ou alcoxy en C₁₋₄; un résidu 2-triazinyle ou 1-benzotriazolyle non substitué ou portant un substituant halogéno ou hydroxy;
R₆ représente un résidu halogéno, nitro, alkyle en C₁₋₄, halogénoalkyle en C₁₋₄, amino, mono-(alkyle en C₁₋₄)-amino; di-(alkyle en C₁₋₄)-amino ou COR₁₁;
n vaut 0, 1, 2, 3 ou 4;
R₇ représente un groupe alkyle en C₁₋₈; halogénoalkyle en C₁₋₈; phényle ou phényl-(alkyle en C₁₋₄) non substitué ou portant, sur le noyau phényle, un substituant halogéno, alkyle en C₁₋₄, halogénoalkyle en C₁₋₄ ou alcoxy en C₁₋₄;
R₈ représente un atome d'hydrogène, un résidu alkyle en C₁₋₈, halogénoalkyle en C₁₋₈, phényle ou phényl-(alkyle en C₁₋₄) non substitué ou portant, sur le noyau phényle, un substituant halogéno, alkyle en C₁₋₄, halogénoalkyle en C₁₋₄ ou alcoxy en C₁₋₄;
R₉ et R₁₀ représentent, indépendamment l'un de l'autre, un atome d'hydrogène ou un groupe alkyle en C₁₋₈, ou
R₉ et R₁₀ forment avec l'atome d'azote auquel ils sont liés un résidu pyrrolidine, pipéridine, morpholine, thiomorpholine ou pipérazine non substitué ou portant un substituant alkyle en C₁₋₄;
R₁₁ représente un atome d'hydrogène; un résidu hydroxy; alkyle en C₁₋₈, alcoxy en C₁₋₈, halogénoalkyle en C₁₋₈; un résidu phényle, phényl-(alkyle en C₁₋₄) ou phényle-(alcoxy en C₁₋₄) non substitué ou portant, sur le noyau phényle, un substituant halogéno, alkyle en C₁₋₄, halogénoalkyle en C₁₋₄ ou alcoxy en C₁₋₄;
(b) comme agents tensioactifs anioniques,
(ba) des alcanes sulfatés
(bb) des alcanesulfonates
(bc) des carboxylates sulfonés, ou
(bd) des carboxylates sulfatés, ou
(be) des esters acides, ou les sels correspondants, de produits d'addition d'oxyde d'alkylène et
(c) un agent épaississant.

2. Formulation liquides conformes à la revendication 1, caractérisées en ce que l'on utilise comme agent chromogène un fluorane de formule (1) dans laquelle
R₁ représente un atome d'hydrogène ou un groupe alkyle en C₁₋₄,
R₂ et R₃ représentent, indépendamment l'un de l'autre, un atome d'hydrogène ou un groupe alkyle en C₁₋₅, ou
R₂ et R₃ forment avec l'atome d'azote auquel ils sont liés, un noyau pyrrolidine ou pipéridine non substitué ou portant un substituant alkyle en C₁₋₄;
R₄ représente un atome d'hydrogène ou un résidu alkyle en C₁₋₄;
R₅ représente un groupe nitro; SO₂R₇; SO₂OR₈; SO₂NR₉R₁₀; COR₁₁; CONR₉R₁₀; halogénoalkyle en C₁₋₄; un résidu phényle, phénylamino ou phényl-(alkyle en C₁₋₄)-amino non substitué ou portant, sur le noyau phényle, un substituant alkyle en C₁₋₄, halogénoalkyle en C₁₋₄ ou alcoxy en C₁₋₄;
n vaut 0, 1, 2, 3 ou 4;
R₆, lorsque n vaut 1, 2, 3 ou 4, représente un résidu halogéno; lorsque n vaut 1 ou 2, représente un résidu alkyle en C₁₋₄ ou halogénoalkyle en C₁₋₄, ou lorsque n vaut 1 représente un résidu nitro, COR₁₁, amino, mono-(alkyle en C₁₋₄)-amino ou di-(alkyle en C₁₋₄)-amino;
R₇ représente un groupe alkyle en C₁₋₄; halogénoalkyle en C₁₋₄; phényle ou phényl-(alkyle en C₁₋₂) non substitué ou portant, sur le noyau phényle, un substituant halogéno, alkyle en C₁₋₄, halogénoalkyle en C₁₋₄ ou alcoxy en C₁₋₄;
R₈ représente un atome d'hydrogène, un résidu alkyle en C₁₋₄; halogénoalkyle en C₁₋₄; phényle ou phényl-(alkyle en C₁₋₂) non substitué ou portant, sur le noyau phényle, un substituant halogéno, alkyle en C₁₋₄, halogénoalkyle en C₁₋₄ ou alcoxy en C₁₋₄;
R₉ et R₁₀ représentent, indépendamment l'un de l'autre, un atome d'hydrogène ou un groupe alkyle en C₁₋₈, ou
R₉ et R₁₀ forment avec l'atome d'azote auquel ils sont liés un résidu pyrrolidine ou pipéridine non substitué ou portant un substituant alkyle en C₁₋₄, et
R₁₁ représente un atome d'hydrogène, un résidu alkyle en C₁₋₄, halogénoalkyle en C₁₋₄; alcoxy en C₁₋₄; phényle, phényl-(alkyle en C₁₋₂) ou phényle-(alcoxy en C₁₋₂) non substitué ou portant, sur le noyau phényle, un substituant halogéno, alkyle en C₁₋₄, halogénoalkyle en C₁₋₄ ou alcoxy en C₁₋₄.

3. Formulations liquides conformes à la revendication 1 ou 2, caractérisées en ce que l'on utilise comme agent chromogène un fluorane de formule (1) dans laquelle
R₁ représente un atome d'hydrogène ou un groupe méthyle,
R₂ et R₃ représentent, indépendamment l'un de l'autre, un atome d'hydrogène ou un groupe alkyle en C₁₋₅, ou
R₂ et R₃ forment avec l'atome d'azote auquel ils sont liés, un noyau pyrrolidine ou pipéridine non substitué;
R₄ représente un atome d'hydrogène ou un résidu méthyle;
R₅ représente un groupe nitro; SO₂R₇; SO₂OR₈; SO₂NR₉R₁₀; COR₁₁; CONR₉R₁₀; halogénoalkyle en C₁₋₄; phényle, phényl-(alkyle en C₁₋₄), phénylamino ou phényl-(alkyle en C₁₋₄)-amino non substitué ou portant, sur le noyau phényle, un substituant halogéno, alkyle en C₁₋₄, halogénoalkyle en C₁₋₄ ou alcoxy en C₁₋₄;
n vaut 0, 1, 2, 3 ou 4;
R₆, lorsque n vaut 1, 2, 3 ou 4, représente un résidu halogéno; lorsque n vaut 1 ou 2, représente un résidu méthyle, ou lorsque n vaut 1 représente un résidu nitro, amino, mono-(alkyle en C₁₋₄)-amino ou di-(alkyle en C₁₋₄)-amino;
R₇ représente un groupe alkyle en C₁₋₄ halogénoalkyle en C₁₋₄; phényle non substitué ou portant un substituant halogéno, alkyle en C₁₋₄ ou alcoxy en C₁₋₄;
R₉ et R₁₀ représentent, indépendamment l'un de l'autre, un atome d'hydrogène ou un groupe alkyle en C₁₋₄,
R₁₁ représente un atome d'hydrogène; un résidu alkyle en C₁₋₄; alcoxy en C₁₋₄; un résidu phényle, phényl-(alkyle en C₁₋₂) ou phényl-(alcoxy en C₁₋₂) non substitués ou portant un substituant halogéno, alkyle en C₁₋₄, halogénoalkyle en C₁₋₄ ou alcoxy en C₁₋₄.

4. Formulations liquides conformes à une des revendications 1 à 3 contenant au moins un agent chromogène de formule (1) dans laquelle R₅ représente un groupe COR₁₁.

5. Formulations liquides conformes à la revendication 4, caractérisées en ce que R₁₁ représente un groupe alkyle en C₁₋₄, alcoxy en C₁₋₄ ou phényl-(alcoxy en C₁₋₂).

6. Formulations liquides conformes à une des revendications 1 à 3 contenant au moins un agent chromogène de formule (1) dans laquelle R₅ représente un résidu phényle, phénylamino ou phényl-(alkyle en C₁₋₄)-amino.

7. Formulations liquides conformes à la revendication 1, caractérisées en ce que l'on utilise comme agent chromogène un fluorane de formule (1) dans laquelle R₂ et R₃ représentent, indépendamment l'un de l'autre, un atome d'hydrogène, un groupe alkyle en C₁₋₁₂, cycloalkyle en C₅₋₇, alcényle en C₃₋₅ ou benzyle, ou R₂ et R₃ forment avec l'atome d'azote auquel ils sont liés un résidu hétérocyclique.

8. Formulations liquides conformes à la revendication 7, caractérisées en ce que l'on utilise comme agent chromogène un fluorane de formule (1) dans laquelle R₂ et R₃ représentent indépendamment l'un de l'autre un groupe alkyle en C₁₋₄.

9. Formulations liquides conformes à une des revendications 1 à 8, caractérisées en ce que l'on utilise comme agents tensioactifs (ba) des sels d'esters d'acide sulfurique et d'alcools gras de formule
(2) R₁₂-O-SO₃-M₁
où R₁₂ représente un résidu alkyle en C₆ à C₁₈ et M₁ est un métal alcalin.

10. Formulations liquides conformes à une des revendications 1 à 8, caractérisées en ce que l'on utilise comme agents tensioactifs anioniques (bb) des alcane-sulfonates secondaires ayant des chaînes longues de 14 à 8 atomes de carbone.

11. Formulations liquides conformes à une des revendications 14 à 18, caractérisées en ce que l'on utilise comme agents tensioactifs anioniques (bc) des composés de formule 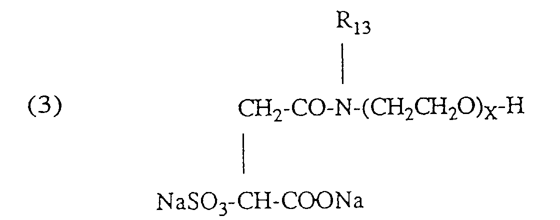 dans laquelle
R₁₃ représente un atome d'hydrogène ou un groupe (CH₂CH₂O)ₓH et
x vaut entre 1 et 10.

12. Formulations liquides conformes à une des revendications 1 à 8, caractérisées en ce que l'on utilise comme agent tensioactif (bd) des sels de métaux alcalins, d'ammonium ou d'amine des sulfates d'acides gras comportant de 10 à 22 atomes de carbone.

13. Formulations liquides conformes à une des revendications 1 à 8, caractérisées en ce que l'on utilise comme agent tensioactif anionique (be) un composé de formule 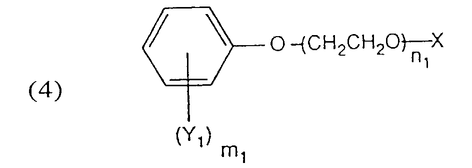 dans laquelle
Y₁ représente un groupe alkyle en C₄₋₁₂, phényle, tolyle, phényl-(alkyle en C₁₋₃) ou tolyl-(alkyle en C₁₋₃),
X₁ représente le résidu dérivé d'acide sulfurique ou d'acide orthophosphorique, et
m₁ vaut entre 1 et 3 et
n₁ entre 4 et 40.

14. Formulations liquides conformes à la revendication 13, caractérisées en ce que l'on utilise comme agent tensioactif (be) un composé de formule 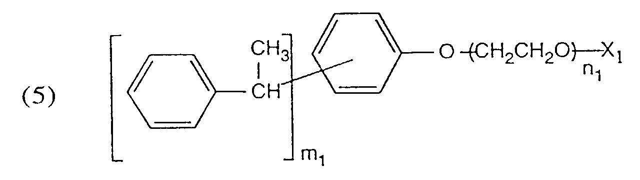 dans laquelle
X₁ représente un résidu dérivé d'acide sulfurique ou d'acide orthophosphorique, et
m₁ vaut entre 1 et 3 et
n₁ entre 4 et 40.

15. Formulations liquides conformes à une des revendications 1 à 14, caractérisées en ce que l'on utilise comme agent épaississant
(ca) des polysaccharides modifiés par des groupes ioniques,
(cb) de la cellulose non ionique et ses dérivés,
(cc) des carboxylates synthétiques hydrosolubles,
(cd) du poly(alcool vinylique),
(ce) de la bentonite ou
(cf) des agents tensioactifs non ionogènes.

16. Formulations liquides conformes à la revendications 15, caractérisées en ce que l'on utilise comme agent épaississant des polysaccharides modifiés par des groupes ioniques.

17. Formulations liquides conformes à la revendications 15 ou 16, caractérisées en ce que l'on utilise comme agent épaississant de l'alginate de sodium.

18. Formulations liquides conformes à la revendication 15 ou 16, caractérisées en ce que l'on utilise comme agent épaississant du xanthane.

19. Formulations liquides conformes à la revendication 15, caractérisées en ce que l'on utilise comme composant (cc) du poly(acide acrylique) ayant une masse molaire de 150 000 à 200 000.

20. Formulations liquides conformes à la revendication 15, caractérisées en ce que l'on utilise comme agent épaississant du poly(alcool vinylique) ayant une masse molaire de 15 000 à 1 500 000.

21. Formulations liquides conformes à la revendication 15, caractérisées en ce que l'on utilise comme agent tensioactif non ionogène (cf) un copolymère d'oxyde d'éthylène et d'oxyde de propylène.

22. Formulations liquides conformes à la revendication 15, caractérisées en ce que l'on utilise comme agent tensioactif non ionogène (cf) un produit d'addition d'oxyde d'alkylène sur des alcools aliphatiques mono- à hexavalents saturés ou insaturés.

23. Formulations liquides conformes à la revendication 1, caractérisées en ce qu'elles contiennent
(a) un agent chromogène de formule (1)
(b) un sel alcalin d'un ester sulfate d'un acide gras en C₁₀ - C₁₈, et
(c) un polysaccharide modifié par des groupes ioniques.

24. Formulations liquides conformes à la revendication 23, caractérisées en ce qu'elles contiennent
(a) un agent chromogène de formule (1) où R₂ et R₃ représentent, indépendamment l'un de l'autre, un résidu alkyle en C₁₋₄, et R₅ représente un résidu phényle, phénylamino ou phényl-(alkyle en C₁₋₄)-amino;
(b) le sel sodique d'un ester sulfate d'un acide gras en C₁₀ - ₁₈, et
(c) de l'alginate de sodium.

25. Formulations liquides conformes à la revendication 23, caractérisées en ce qu'elles contiennent
(a) un agent chromogène de formule (1), où R₂ et R₃ représentent, indépendamment l'un de l'autre, un résidu alkyle en C₁₋₄, et R₅ représente un résidu phényle, phénylamino ou phényl(alkyle en C₁₋₄)-amino,
(b) un agent tensioactif de formule (3), et
(c) de l'alginate de sodium.

26. Formulations liquides conformes à la revendication 1, caractérisées en ce qu'elles contiennent
(a) un agent chromogène de formule (1),
(b) un sel alcalin d'un ester sulfate d'un acide gras en C₁₀-₁₈ et
(c) un agent tensioactif non ionogène difficilement soluble dans l'eau.

27. Formulations liquides conformes à une des revendications 1 à 26 caractérisées en ce qu'elles contiennent
de 20 à 60 % en poids d'un agent chromogène (a),
de 1 à 15 % en poids d'un agent tensioactif anionique (b),
de 0,1 à 5 % en poids d'un agent épaississant (c) et
une quantité suffisante d'eau pour compléter à 100 %.

28. Procédé pour la préparation des formulations liquides conformes à une des revendications 1 à 27, caractérisé en ce que l'on broie l'agent chromogène (a) avec l'agent tensioactif anionique en présence d'eau et en ce que l'on ajoute ensuite l'agent épaississant (c).

29. Utilisation des formulations liquides conformes à une des revendications 1 à 27 en tant que matériau d'enregistrement thermosensible.
